(19) Europäisches Patentamt
European Patent Office
Office européen des brevets

(11) Veröffentlichungsnummer: **0 474 644 B1**

(12) **EUROPÄISCHE PATENTSCHRIFT**

(45) Veröffentlichungstag der Patentschrift: **28.09.94**

(51) Int. Cl.5: **C07C 67/29**, C07C 69/52, C07C 69/24, C07C 69/30

(21) Anmeldenummer: **90906195.4**

(22) Anmeldetag: **19.04.90**

(86) Internationale Anmeldenummer: **PCT/EP90/00630**

(87) Internationale Veröffentlichungsnummer: **WO 90/13533 (15.11.90 90/26)**

(54) **VERWENDUNG VON CALCINIERTEN HYDROTALCITEN ALS KATALYSATOREN FÜR DIE ETHOXYLIERUNG BZW. PROPOXYLIERUNG VON FETTSÄUREESTERN.**

(30) Priorität: **28.04.89 DE 3914131**

(43) Veröffentlichungstag der Anmeldung: **18.03.92 Patentblatt 92/12**

(45) Bekanntmachung des Hinweises auf die Patenterteilung: **28.09.94 Patentblatt 94/39**

(84) Benannte Vertragsstaaten: **DE FR IT**

(56) Entgegenhaltungen:
**EP-A- 0 339 426**
**FR-A- 2 251 542**

**CHEMICAL ABSTRACTS, Band 95, Nr. 11, 14. September 1981, Columbus, Ohio, US, Seite 590, Zusammenfassung Nr. 97099m; & JP-A-8136431**

(73) Patentinhaber: **Henkel Kommanditgesellschaft auf Aktien**

**D-40191 Düsseldorf (DE)**

(72) Erfinder: **BEHLER, Ansgar**
**Siegfriedstrasse 80**
**D-4250 Bottrop (DE)**
Erfinder: **RATHS, Hans-Christian, Dr.**
**Wiener Neustädter Strasse 95**
**D-4019 Monheim 2 (DE)**
Erfinder: **FRIEDRICH, Klaus**
**Marconistrasse 13**
**D-4000 Düsseldorf 13 (DE)**
Erfinder: **HERRMANN, Klaus**
**Köpenicher Strasse 33**
**D-4019 Monheim (DE)**

**Beschreibung**

Die Erfindung betrifft die Verwendung von calcinierten Hydrotalciten als Katalysatoren für die Ethoxylierung bzw. Propoxylierung von Fettsäureestern, welche aus der von Estern von gegebenenfalls hydroxy-substituierten Fettsäuren mit 8 bis 22 Kohlenstoffatomen mit Monoalkanolen mit 1 bis 22 Kohlenstoffatomen sowie von Partialestern und Vollestern von gegebenenfalls hydroxy-substituierten Fettsäuren mit 8 bis 22 Kohlenstoffatomen mit Polyolen mit 2 bis 12 Kohlenstoffatomen und 2 bis 6 Hydroxylgruppen gebildeten Gruppe ausgewählt sind.

Hydrotalcit ist ein natürliches Mineral mit der Idealformel

$$Mg_6 Al_2(OH)_{16} CO_3 . 4H_2O$$

dessen Struktur von derjenigen des Brucits ($Mg(OH)_2$) abgeleitet ist. Brucit kristallisiert in einer Schichtstruktur mit den Metallionen in Oktaederlücken zwischen zwei Schichten aus dichtgepackten Hydroxylionen, wobei nur jede zweite Schicht der Oktaederlücken besetzt ist. Im Hydrotalcit sind einige Magnesiumionen durch Aluminiumionen ersetzt, wodurch das Schichtpaket eine positive Ladung erhält. Diese wird durch die Anionen ausgeglichen, die sich zusammen mit zeolithischen Kristallwasser in den Zwischenschichten befinden. Der Schichtaufbau wird in dem Röntgenpulverdiagramm deutlich (ASTM-Karte Nr.14-191), das zur Charakterisierung herangezogen werden kann.

Es sind auch synthetische Hydrotalcite bekannt, die z.B. in den DE-C 1 592 126, DE-A 3 346 943, DE-A 3 306 822 und EP-A 0 207 811 beschrieben sind.

In natürlichen und synthetischen Produkten kann das $Mg^{2+}:Al^{3+}$-Verhältnis zwischen etwa 1 und 5 variieren. Auch das Verhältnis von $OH^-:CO_3^{2-}$ kann schwanken. Natürliche und synthetische Hydrotalcite können durch die allgemeine Formel I

$$Mg_x Al(OH)_y(CO_3)_z . n H_2O \qquad (I)$$

näherungsweise beschrieben werden, wobei die Bedingungen $1 < x < 5$, $y > z$, $(y + 2z) = 2x + 3$ und $0 < n < 10$ gelten. Unterschiede in der Zusammensetzung der Hydrotalcite, insbesondere bezüglich des Wassergehaltes, führen zu Linienverschiebungen im Röntgenbeugungsdiagramm.

Natürliche oder synthetische Hydrotalcite geben beim Erhitzen bzw. Calcinieren kontinuierlich Wasser ab. Die Entwässerung ist bei 200°C vollständig, wobei durch Röntgenbeugung nachgewiesen werden konnte, daß die Struktur des Hydrotalcits noch erhalten geblieben ist. Die weitere Temperaturerhöhung führt unter Abspaltung von Hydroxylgruppen (als Wasser) und von Kohlendioxid zum Abbau der Struktur. Natürliche und nach verschiedenen Verfahren, z.B. gemäß den obigen Veröffentlichungen, hergestellte synthetische Hydrotalcite zeigen bei der Calcinierung ein generell ähnliches Verhalten.

Calcinierte Hydrotalcite sind bereits für verschiedene Zwecke eingesetzt worden, z.B. als Absorptionsmittel sowie bei Umsetzungen von Alkylenoxiden mit Alkylacetaten zur Herstellung von Mono-, Di- und Triethylenglykolethylether-acetaten, vgl. JP-A 56/36 431, referiert in C.A.95(11)97 099m (1981).

Für Polyalkoxylate ist eine enge Bandbreite des Polyalkoxylierungsgrades von besonderer Bedeutung, vgl. JAOCS, Vol. 63, 691 - 695 (1986), und HAPPI, 52 - 54 (1986).

Es wurde nun gefunden, daß man unter erfindungsgemäßer Verwendung calcinierter Hydrotalcite als Katalysatoren Fettsäureester der eingangs genannten Art bei kurzen Reaktionszeiten mit hohen Ausbeuten polyalkoxylieren kann und die Reaktionsprodukte mit einer engeren Bandbreite bzw. Homologenverteilung als bei der Verwendung des üblicherweise als Katalysator eingesetzten Natriummethylats erhalten werden können.

Für die Zwecke der Erfindung eignen sich sämtliche der durch Calcinierung aus den eingangs genannten natürlichen und/oder synthetischen Hydrotalciten erhältlichen Katalysatoren; bevorzugt sind Hydrotalcite, die vor der Calcinierung die allgemeine Formel I

$$Mg_x Al(OH)_y(CO_3)_z . n H_2O \qquad (I)$$

mit den oben angegebenen Bedingungen für x, y, z und n aufweisen; besonders bevorzugt sind Werte für x von 1,8 bis 3.

Die erfindungsgemäß eingesetzten calcinierten Hydrotalcite weisen den Vorteil auf, daß sie in das Reaktionsgemisch der Alkoxylierung leicht eingearbeitet werden und wegen ihrer Unlöslichkeit in dem Reaktionsgemisch durch einfache Maßnahmen wieder abgetrennt werden können. Sie können jedoch auch in den Reaktionsgemisch verbleiben, wenn ihre Anwesenheit bei der Weiterverwendung der Reaktionsprodukte nicht stört.

Beispiele für erfindungsgemäß unter Verwendung von calcinierten Hydrotalciten alkoxylierbare Fettsäureester der eingangs genannten Art sind im folgenden aufgeführt, wobei zunächst die Fettsäurekomponente der Fettsäureester und anschließend die Alkoholkomponente derselben getrennt aufgezählt werden.

Fettsäuren:

Fettsäuren mit 8 bis 22 Kohlenstoffatomen natürlicher oder synthetischer Herkunft, insbesondere geradkettige, gesättigte oder ungesättigte Fettsäuren einschließlich technischer Gemische derselben, wie sie durch Fettspaltung aus tierischen und/oder pflanzlichen Fetten und Ölen zugänglich sind, z.B. aus Kokosöl, Palmkernöl, Palmöl, Soyaöl, Sonnenblumenöl, Rüböl, Baumwollsaatöl, Fischöl, Rindertalg und Schweineschmalz; spezielle Beispiele sind Capryl-, Caprin-, Laurin-, Laurolein-, Myristin-, Myristolein-, Palmitin-, Palmitolein-, Öl-, Elaidin-, Arachin-, Gadolein-, Behen-, Brassidin- und Erucasäure; weiterhin methylverzweigte, gesättigte und ungesättigte Fettsäuren mit 10 bis 22 Kohlenstoffatomen, die bei der Dimerisierung von den entsprechenden ungesättigten Fettsäuren als Nebenprodukte entstehen.

Hydroxyfettsäuren:

Natürliche oder synthetische Hydroxyfettsäuren, insbesondere mit 16 bis 22 Kohlenstoffatomen, z.B. Ricinolsäure oder 12-Hydroxystearinsäure.

Alkanole:

Gesättigte oder ungesättigte Monoalkanole, insbesondere Hydrierungsprodukte der oben genannten geradkettigen, gesättigten oder ungesättigten Fettsäuren bzw. Derivate derselben wie Methylester oder Glyceride; aliphatische oder cyclische Alkanole mit 1 bis 6 Kohlenstoffatomen, z.B. Methanol, Ethanol, Propanol, Butanol, Hexanol und Cyclohexanol; einschließlich der von den vorgenannten Monoalkanolen abgeleiteten Guerbet-Alkohole.

Polyole:

Ethylenglykol, 1,2-Propylenglykol, 1,2-Butylenglykol, Neopentylglykol, Glycerin, Diglycerin, Triglycerin, Tetraglycerin, Trimethylolpropan, Di-trimethylolpropan, Pentaerythrit, Di-Pentaerythrit, und Zuckeralkohole, insbesondere Sorbitan.

Wie bereits eingangs ausgeführt wurde, können im Falle von Estern der oben genannten Fettsäuren mit den vorgenannten Polyolen diese auch als Partialester bzw. Partialesterenthaltende technische Estergemische, insbesondere in Form von Glyceriden, vorliegen.

Die Struktur der erfindungsgemäß erhaltenen ethoxylierten bzw. propoxylierten Fettsäureester ist nicht immer eindeutig feststellbar. Monoalkanolester und Polyolvollester von Fettsäuren reagieren - mit hoher Wahrscheinlichkeit unter Einschub von Ethylenoxy- und/oder Propylenoxy-Einheiten in die Esterbindung - zu praktisch hydroxylgruppenfreien Endprodukten. Die Struktur der hydroxylgruppenhaltigen Produkte, die bei der Reaktion von Polyolpartialestern von Fettsäuren oder Monoalkanolestern von Hydroxyfettsäuren mit Ethylenoxid und/oder Propylenoxid entstehen, ist nicht bekannt; hier sind auch Reaktionen an den freien OH-Gruppen denkbar, und zwar insbesondere bei freien, primären OH-Gruppen.

Die erfindungsgemäß unter Verwendung von calcinierten Hydrotalciten herzustellenden Derivate sind handelsübliche Produkte, so daß sich einen nähere Erläuterung erübrigt. Typische Vertreter dieser Derivate sind beispielsweise Anlagerungsprodukte von 41 mol Ethylenoxid an 1 mol Ricinusöl, Anlagerungsprodukte von 25 mol Ethylenoxid an 1 mol gehärtetes Ricinusöl, Anlagerungsprodukte von 7 Gew.-Teilen Ethylenoxid an 10 Gew.-Teile eines Palmitinsäure-/Stearinsäuremono-/diglyceridgemisches mit einem Anteil von 40 bis 45 Gew.-% Monoglycerid und Anlagerungsprodukte von 20 mol Ethylenoxid an 1 mol Sorbitanmonostearat.

Gemäß einer weiteren vorteilhaften Ausführungform der Erfindung setzt man die calcinierten Hydrotalcite in einer Menge von 0,1 bis 2 Gew.-%, bezogen auf das Endprodukt der Ethoxylierung bzw. Propoxylierung, den Reaktionsgemischen zu.

Die erfindungsgemäß einzusetzenden calcinierten Hydrotalcite können aus den natürlichen oder synthetischen Hydrotalciten durch mehrstündiges Erhitzen auf Temperaturen von über 100 °C erhalten werden; besonders bevorzugt sind Calcinierungstemperaturen von 400 bis 600 °C.

Die Erfindung wird im folgenden anhand bevorzugter Ausführungsbeispiele und eines Vergleichsbeispiels näher erläutert.

Beispiel 1.

Ricinusöl + 1,4 mol Ethylenoxid.

Ein handelüblicher synthetischer Hydrotalcit wurde 4 h bei 500 °C calciniert.

Zur Umsetzung eines handelsüblichen Ricinusöls mit Ethylenoxid (Molverhältnis 1 : 1,4) wurde das Ricinusöl in einem Druckreaktor vorgelegt und mit 0,5 Gew.-%, bezogen auf erwartetes Endprodukt, des zuvor erhaltenen calcinierten Hydrotalcits versetzt. Der Reaktor wurde mit Stickstoff gespült und 30 min lang bei einer Temperatur von 100 °C evakuiert. Anschließend wurde die Temperatur auf 165 - 175 °C gesteigert und die gewünschte Menge Ethylenoxid bei einem Druck von 3 bis 5 bar aufgedrückt. Nach Beendigung der Reaktion ließ man 30 min. nachreagieren (Gesamtzeit der Reaktion: 3 h). Nach dem Abfiltrieren von suspendiertem Katalysator erhielt man das gewünschte Reaktionsgemisch mit einer Verseifungszahl von 155 (theor.: 138).

Beispiel 2.

Methyllaurat + 2 mol Ethylenoxid.

Unter den in Beispiel 1 angegebenen Bedingungen erhielt man aus einem handelsüblichen Laurinsäuremethylester und 2 mol Ethylenoxid bei einer Reaktionszeit von 0,75 h das gewünschte Produkt mit einer Verseifungungszahl von 185 (theor.: 185,7).

Beispiel 3.

Rüböl + 3 mol Ethylenoxid.

Unter den in Beispiel 1 angegebenen Bedingungen erhielt man aus einem handelsüblichen Rüböl das oben genannte Ethoxylierungsprodukt mit einer Verseifungszahl von 150 (theor.: 130). Die Gesamtreaktionszeit betrug 2,5 h.

Vergleichsbeispiel.

Das Beispiel 2 wurde wiederholt, wobei anstelle des dort eingesetzten calcinierten Hydrotalcits Natriummethylat als Katalysator verwendet wurde. Die Zusammensetzung des erhaltenen Ethoxylierungsproduktes wurde gaschromatographisch (in Flächenprozent) ermittelt, und mit derjenigen des Produktes des Beispiels 2 verglichen; die Ergebnisse sind in der folgenden Tabelle zusammengefaßt. Die Bezeichnung "EO-Grad" bedeutet die Anzahl der an Methyllaurat angelagerten Ethylenoxidmoleküle, wobei ein Ethoxylierungsgrad von 0 nicht umgesetztes Ausgangsprodukt bedeutet.

Die Tabelle zeigt, daß in dem Produkt des Vergleichsbeispiels die Menge des nicht umgesetzten Ausgangsproduktes um den Faktor 2 höher und die Menge des gewünschten Endproduktes mehr als 3 mal niedriger als in dem Produkt des Beispiels 2 ist.

4

EP 0 474 644 B1

Tabelle

| Homologenverteilung bei der Ethoxylierung von Methyllaurat | | |
|---|---|---|
| EO-Grad | Beispiel 2 | Vergleichsbeispiel |
| | (Flächenprozent) | |
| 0 | 31,3 | 60,0 |
| 1 | 12,1 | 11,2 |
| 2 | 14,1 | 4,2 |
| 3 | 12,2 | 3,3 |
| 4 | 9,6 | 3,0 |
| 5 | 5,8 | 4,2 |
| 6 | 4,3 | 2,8 |
| 7 | 3,2 | 2,6 |
| 8 | 2,4 | 2,3 |
| 9 | 1,8 | 2,0 |
| 10 | 1,4 | 1,7 |
| 11 | 0,9 | 1,2 |
| 12 | 0,6 | 0,9 |

**Patentansprüche**

1. Verwendung von calcinierten Hydrotalciten als Katalysatoren für die Ethoxylierung bzw. Propoxylierung von Fettsäureestern, welche aus der von Estern von gegebenenfalls hydroxy-substituierten Fettsäuren mit 8 bis 22 Kohlenstoffatomen mit Monoalkanolen mit 1 bis 22 Kohlenstoffatomen sowie von Partialestern und Vollestern von gegebenenfalls hydroxy-substituierten Fettsäuren mit 8 bis 22 Kohlenstoffatomen mit Polyolen mit 2 bis 12 Kohlenstoffatomen und 2 bis 6 Hydroxylgruppen gebildeten Gruppe ausgewählt sind.

2. Verwendung nach Anspruch 1, dadurch gekennzeichnet, daß man gesättigte oder ungesättigte Fettsäure-$C_1$-$C_4$-alkylester ethoxyliert bzw. propoxyliert.

3. Verwendung nach Anspruch 1 oder 2, dadurch gekennzeichnet, daß man Glyceride von gegebenenfalls monohydroxysubstituierten, gesättigten oder ungesättigten Fettsäuren ethoxyliert bzw. propoxyliert.

4. Verwendung nach mindestens einem der Ansprüche 1 bis 3, dadurch gekennzeichnet, daß die Hydrotalcite vor dem Calcinieren eine Zusammensetzung der Formel I

$$Mg_xAl(OH)_y(CO_3)_z \cdot n\ H_2O \qquad (I)$$

aufweisen, in der die Bedingungen $1 < x < 5$, $y > z$, $(y + 2z) = 2x + 3$ und $0 < n < 10$ gelten.

5. Verwendung nach mindestens einem der Ansprüche 1 bis 4, dadurch gekennzeichnet, daß für die Hydrotalcite der allgemeinen Formel I

$$Mg_xAl(OH)_y(CO_3)_z \cdot n\ H_2O$$

x eine Zahl von 1,8 bis 3 ist und y, z sowie n wie oben definiert sind.

6. Verwendung nach mindestens einem der Ansprüche 1 bis 5, dadurch gekennzeichnet, daß die Hydrotalcite bei Temperaturen zwischen 400 und 600°C calciniert wurden.

7. Verwendung nach mindestens einem der Ansprüche 1 bis 6, dadurch gekennzeichnet daß man die calcinierten Hydrotalcite in einer Menge von 0,1 bis 2 Gew.-%, bezogen auf das Endprodukt der Ethoxylierung bzw. Propoxylierung, einsetzt.

5

**Claims**

1. The use of a calcined hydrotalcite as catalyst for the ethoxylation or propoxylation of fatty acid esters selected from the group formed by esters of optionally hydroxy-substituted fatty acids having 8 to 22 carbon atoms with monoalkanols having 1 to 22 carbon atoms, and by partial esters and full esters of optionally hydroxy-substituted fatty acids having 8 to 22 carbon atoms with polyols having 2 to 12 carbon atoms and 2 to 6 hydroxyl groups.

2. The use as claimed in claim 1, wherein saturated or unsaturated fatty acid $C_1$-$C_4$-alkyl esters are ethoxylated or propoxylated.

3. The use as claimed in claim 1 or 2, wherein glycerides of optionally monohydroxy-substituted, saturated or unsaturated fatty acids are ethoxylated or propoxylated.

4. The use as claimed in at least one of claims 1 to 3, wherein the hydrotalcite, before calcination, has a composition of the formula I

$$Mg_xAl(OH)_y(CO_3)_z \cdot n\ H_2O \qquad (I)$$

in which the conditions $1 < x < 5$, $y > z$, $(y + 2z) = 2x + 3$ and $0 < n < 10$ apply.

5. The use as claimed in at least one of claims 1 to 4, wherein, for the hydrotalcite of the general formula I

$$Mg_xAl(OH)_y(CO_3)_z \cdot n\ H_2O$$

x is a number from 1.8 to 3, and y, z and n are as defined above.

6. The use as claimed in at least one of claims 1 to 5, wherein the hydrotalcite had been calcined at a temperature between 400 and 600°C.

7. The use as claimed in at least one of claims 1 to 6, wherein the calcined hydrotalcite is employed in an amount of from 0.1 to 2% by weight, relative to the end product of the ethoxylation or propoxylation.

**Revendications**

1. Utilisation d'hydrotalcites calcinées comme catalyseurs pour l'éthoxylation ou la propoxylation d'esters d'acide gras, qui sont choisis dans le groupe formé d'esters d'acide gras éventuellement substitués par un hydroxy, ayant de 8 à 22 atomes de carbone avec des monoalcanols ayant de 1 à 22 atomes de carbone ainsi que des esters partiels et les esters complets d'acide gras éventuellement substitués par un hydroxy, ayant de 8 à 22 atomes de carbone avec des polyols ayant de 2 à 12 atomes de carbone et de 2 à 6 groupes hydroxyle.

2. Utilisation selon la revendication 1, caractérisée en ce que l'on éthoxyle ou propoxyle des esters d'alcoyle en $C_1$ à $C_4$ d'acides gras, saturés ou non saturés.

3. Utilisation selon la revendication 1 ou 2, caractérisée en ce que l'on éthoxyle ou propoxyle des glycérides d'acides gras, saturés ou non saturés, le cas échéant substitués par un monohydroxy.

4. Utilisation selon au moins une des revendications 1 à 3, caractérisée en ce que les hydrotalcites avant la calcination possèdent une composition de formule I

$$Mg_xAl(OH)_y(CO_3)_z \cdot n\ H_2O \qquad (I)$$

dans laquelle les conditions $1 < x < 5$, $y > z$, $(y + 2z) = 2x + 3$ et $0 < n < 10$ sont valables.

5. Utilisation selon au moins une des revendications 1 à 4, caractérisée en ce que pour les hydrotalcites de formule générale I

$Mg_xAl(OH)_y(CO_3)_z \cdot n\ H_2O$

x est un nombre allant de 1,8 à 3 et y, z ainsi que n sont définis comme ci-dessus.

6. Utilisation selon au moins une des revendications 1 à 5, caractérisée en ce que les hydrotalcites ont été calcinées à des températures comprises entre 400 et 600 °C.

7. Utilisation selon au moins une des revendications 1 à 6, caractérisée en ce que l'on met en oeuvre les hydrotalcites calcinées en quantité allant de 0,1 à 2 % en poids, rapporté au produit final de l'éthoxylation ou de la propoxylation.